# EUROPEAN PATENT APPLICATION

(11) **EP 4 181 058 A1**
(43) Date of publication of application: **17.05.2023**
(21) Application number: 21218341.2
(22) Date of filing: 30.12.2021
(51) Int. Cl.: G06T 7/00, A61B 6/00

(54) **TIME-RESOLVED ANGIOGRAPHY**

(30) Priority: 10.11.2021 US 202163277740 P
(71) Applicant: Koninklijke Philips N.V., 5656 AG Eindhoven (NL)
(72) Inventor: SALEHI, Leili, Eindhoven (NL); SINHA, Ayushi, Eindhoven (NL); ERKAMP, Ramon Quido, Eindhoven (NL); TOPOREK, Grzegorz Andrzej, Eindhoven (NL); PANSE, Ashish Sattyavrat, Eindhoven (NL)
(74) Representative: Philips Intellectual Property & Standards

(57) **Abstract**

A computer-implemented method of providing a temporal sequence of 3D angiographic images (110) representing a flow of a contrast agent through a region of interest (120), is provided. The method includes: inputting (S130) volumetric image data (130a, 130b), and a temporal sequence of 2D angiographic images (140) into a neural network (NN1); and generating (S140) the predicted temporal sequence of 3D angiographic images (110) representing the flow of the contrast agent through the region of interest (120) in response to the inputting.

## Description

### TECHNICAL FIELD

The present disclosure relates to providing a temporal sequence of 3D angiographic images representing a flow of a contrast agent through a region of interest. A computer-implemented method, a computer program product, and a system, are disclosed.

### BACKGROUND

Time-resolved angiographic imaging is often used in clinical investigations. As compared to static angiographic images, the temporal sequences of angiographic images generated by time-resolved angiographic imaging provide information on the flow of a contrast agent through the vasculature. In turn, the flow information enables an improved understanding of physiological processes in the anatomy. However, the benefits of time-resolved angiographic imaging are achieved at the expense of increased X-ray dose to a patient.

Time-resolved 2D angiographic imaging, and time-resolved 3D angiographic imaging, are both used in clinical investigations. As compared to time-resolved 2D angiographic imaging, the structural information provided by time-resolved 3D angiographic imaging enables a deeper understanding of the flow of a contrast agent in a region of interest. The use of time-resolved 3D angiographic imaging may also improve workflow. For example, the use of time-resolved 3D angiographic imaging obviates the need to accurately align an imaging system with a region of interest in order to provide a desired view of the region of interest. Time-resolved 3D angiographic images may be displayed in 3D, or as synthetic 2D projections from any desired viewing direction in order to provide one or more concurrent views of the region of interest.

Historically, time-resolved 3D angiographic images often had reduced image quality as compared to time-resolved 2D angiographic images due to their increased demand on image processing, and also the need to limit X-ray dose to a patient. More recently, angiographic imaging techniques such as digital subtraction angiography "DSA" have been introduced that provide time-resolved 3D angiographic images with a similar image quality and X-ray dose as time-resolved 2D angiographic images.

However, there remain challenges to using 3D angiographic imaging for some clinical investigations, including for example the potential for a 3D angiographic imaging system to restrict access to a patient, the higher cost of a 3D angiographic imaging system, and the need to provide high quality 3D angiographic images with low X-ray dose and without increasing acquisition time.

### SUMMARY

According to one aspect of the present disclosure, a computer-implemented method of providing a temporal sequence of 3D angiographic images representing a flow of a contrast agent through a region of interest, is provided. The method includes:
receiving volumetric image data representing the region of interest;
receiving a temporal sequence of 2D angiographic images representing the flow of the contrast agent through the region of interest;
inputting the volumetric image data, and the temporal sequence of 2D angiographic images into a neural network; and
in response to the inputting, generating the predicted temporal sequence of 3D angiographic images representing the flow of the contrast agent through the region of interest; and
wherein the neural network is trained to predict the temporal sequence of 3D angiographic images from the temporal sequence of 2D angiographic images, and to constrain the predicted temporal sequence of 3D angiographic images by the volumetric image data.

Since the predicted temporal sequence of 3D angiographic images is provided by a neural network from a temporal sequence of 2D angiographic images, the method provides the benefits of temporal sequences of 3D angiographic images, including structural information that enables a deeper understanding of the flow of a contrast agent in a region of interest, without the drawbacks of needing to accurately align an imaging system with the anatomy, and restricted access to the region of interest.

Further aspects, features, and advantages of the present disclosure will become apparent from the following description of examples, which is made with reference to the accompanying drawings.

### BRIEF DESCRIPTION OF THE DRAWINGS

Fig. 1 is a flowchart illustrating an example of a method of providing a temporal sequence of 3D angiographic images representing a flow of a contrast agent through a region of interest, in accordance with some aspects of the present disclosure.
Fig. 2 is a schematic diagram illustrating an example of a system for providing a temporal sequence of 3D angiographic images 110 representing a flow of a contrast agent through a region of interest 120, in accordance with some aspects of the present disclosure.
Fig. 3 is a schematic diagram illustrating a first example of a method of providing a temporal sequence of 3D angiographic images 110 representing a flow of a contrast agent through a region of interest 120, in accordance with some aspects of the present disclosure.
Fig. 4 is a schematic diagram illustrating a second example of a method of providing a temporal sequence of 3D angiographic images 110 representing a flow of a contrast agent through a region of interest 120, in accordance with some aspects of the present disclosure.
Fig. 5 is a schematic diagram illustrating an orientation of an X-ray source 150b and an X-ray detector 150a of an X-ray imaging system 150 respective a region of interest 120 in a patient, including a rotational angle a of a central ray of the projection X-ray imaging system 150 around a longitudinal axis of a patient, in accordance with some aspects of the present disclosure.
Fig. 6 is a schematic diagram illustrating an orientation of an X-ray source 150b and an X-ray detector 150a of an X-ray imaging system 150 respective a region of interest 120 in a patient, including a tilt angle b of a central ray of the projection X-ray imaging system 150 with respect to a cranial-caudal axis of a patient, in accordance with some aspects of the present disclosure.
Fig. 7 is a flowchart illustrating a first example of a method of training a neural network to provide a temporal sequence of 3D angiographic images representing a flow of a contrast agent through a region of interest, in accordance with some aspects of the present disclosure.
Fig. 8 is a flowchart illustrating a second example of a method of training a neural network to provide a temporal sequence of 3D angiographic images representing a flow of a contrast agent through a region of interest, in accordance with some aspects of the present disclosure.
Fig. 9 is a schematic diagram illustrating a first example of a method of training a neural network to provide a temporal sequence of 3D angiographic images representing a flow of a contrast agent through a region of interest, in accordance with some aspects of the present disclosure.

### DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, computer-implemented methods are described that involve providing a temporal sequence of 3D angiographic images representing a flow of a contrast agent through a region of interest. Reference is made to an example region of interest in the form of a cerebral aneurism. However, it is to be appreciated that this region of interest serves only as an example, and that the methods disclosed herein may also be used to provide temporal sequences of 3D angiographic images representing a flow of a contrast agent through other regions of interest in the vasculature. In general, the region of interest may be anywhere in the vasculature.

Reference is also made herein to an example treatment procedure in the form of an aneurism coiling procedure. In this procedure, coils of wire are inserted into a sac of the aneurism in order to reduce blood flow and thereby enable the blood inside the aneurysm to coagulate. However, it is to be appreciated that this treatment procedure serves only as an example, and that the methods disclosed herein may also be used in other treatment procedures. The methods may be used in treatment procedures such as coronary artery angioplasty, cerebral endovascular thrombectomy, and tumor embolization, for example. It is also to be appreciated that the methods disclosed herein may be used in clinical investigations in general.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

As mentioned above time-resolved angiographic imaging is often used in clinical investigations. Time-resolved 2D angiographic imaging, and time-resolved 3D angiographic imaging, are both used in clinical investigations. As compared to time-resolved 2D angiographic imaging, the structural information provided by time-resolved 3D angiographic imaging enables a deeper understanding of the flow of a contrast agent in a region of interest. Recently, angiographic imaging techniques such as digital subtraction angiography "DSA" have been introduced that provide time-resolved 3D angiographic images with a similar image quality and X-ray dose as time-resolved 2D angiographic images. However, there remain challenges to using 3D angiographic imaging for some clinical investigations, including for example the potential for a 3D angiographic imaging system to restrict access to a patient, the higher cost of a 3D angiographic imaging system, and the need to provide high quality 3D angiographic images with low X-ray dose and without increasing acquisition time.

By way of an example, aneurism coiling procedures typically involve the insertion of wire coils into a sac of the aneurism. This reduces blood flow, and enables blood inside the aneurysm to coagulate. A goal in aneurism coiling is to fill the aneurysm sac with sufficient coils in order to treat the aneurysm, whilst avoiding overfilling. Prior to the aneurism coiling procedure, a static 2D or a 3D angiographic image of the aneurism is often acquired in order to plan the procedure. During the aneurism coiling procedure, static, or time-resolved 2D angiographic images of the aneurism are often generated using a projection X-ray imaging system in order to monitor the progress of the procedure. In order to detect coil material spilling into the parent vessel, a radiographer typically tries to find an orientation of the projection X-ray imaging system that provides 2D images in which there is a minimal amount of overlap between the sac and the parent vessel, and minimal foreshortening of the parent vessel. However, it can be challenging to provide an optimal view of the aneurism. The orientation of the projection X-ray imaging system may need to be adjusted multiple times in order to provide an optimal view of the aneurism.

Sometimes, a projection X-ray imaging system with multiple source-detector arrangements is used to simultaneously provide 2D angiographic images with different views of the aneurism in order to overcome the limitations of a single view. Such imaging systems are often referred-to as "biplane" X-ray imaging systems. The orientations of both of these source-detector arrangements may need to be adjusted several times in order to provide the desired optimal views. Moreover, as the coiling procedure progresses, the optimal view of the aneurism may change. As coils are inserted into the aneurism, the blood flow changes both within the aneurism and in the parent vessel. For instance, before the aneurism coiling procedure begins, blood flows into the aneurism from the portion of the parent vessel proximal to the aneurysm and, consequently, blood flow into the portion of the parent vessel distal to the aneurysm is slow. As the aneurism coiling procedure progresses, less blood flows into the aneurism and blood flow into the portion of the parent vessel distal to the aneurism returns to normal speed. Also, the aneurism can be obscured by the inserted coils. Consequently, it may be necessary to re-adjust the orientation of the projection X-ray imaging system multiple times during an aneurism coiling procedure, which hampers workflow. Other examples of procedures in which blood flow changes during the procedure include atherectomy (i.e. plaque or calcification removal from blood vessels) and balloon angioplasty (i.e. the widening of blood vessels with stenoses).

Providing time-resolved intra-procedural 3D angiographic images of an aneurism might appear to address the challenges of accurately aligning, and re-aligning, the orientation of a 2D X-ray imaging system in order to provide and maintain optimal views of the aneurism. However, the use of a 3D imaging system also incurs challenges. For example, the acquisition of 3D images disrupts procedure workflow because it typically requires procedural staff to step out of the operating room and step back in after the acquisition of the 3D image. Further, in order to generate DSA images, an initial 3D mask image must be acquired, followed by a contrast enhanced 3D image in order to subtract the background anatomy contained in the mask image and only retain vasculature in the time-resolved 3D angiographic image. This significantly increases image acquisition time and demonstrates the potential to further reduce X-ray dose when generating temporal sequences of images. Three-dimensional X-ray imaging systems may also restrict access to a region of interest. Moreover, the expense of a 3D X-ray imaging system is typically higher than that of a 2D X-ray imaging system.

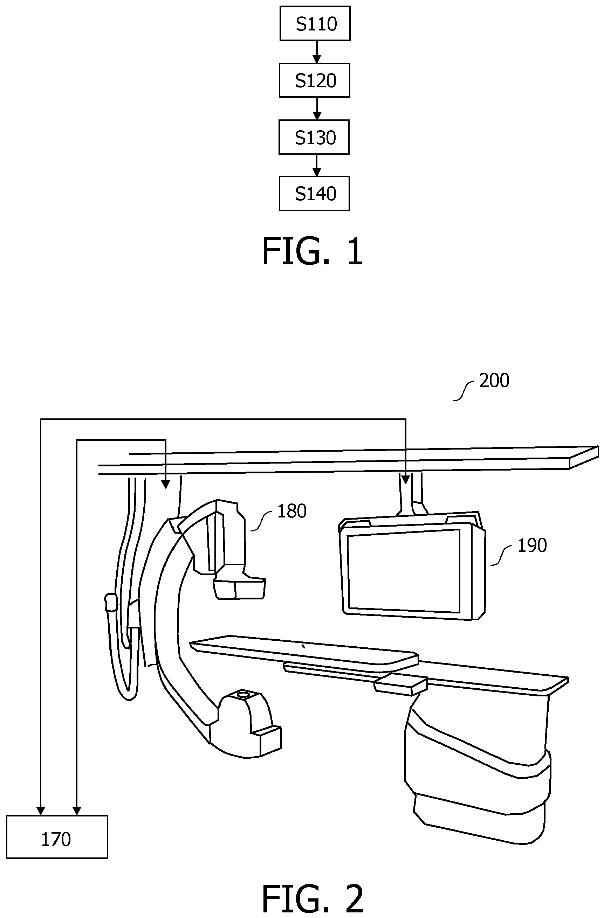

Fig. 1 is a flowchart illustrating an example of a method of providing a temporal sequence of 3D angiographic images representing a flow of a contrast agent through a region of interest, in accordance with some aspects of the present disclosure. With reference to Fig. 1, a computer-implemented method of providing a temporal sequence of 3D angiographic images 110 representing a flow of a contrast agent through a region of interest 120, is provided. The method includes:
receiving S110 volumetric image data 130a, 130b representing the region of interest 120;
receiving S120 a temporal sequence of 2D angiographic images 140 representing the flow of the contrast agent through the region of interest 120;
inputting S130 the volumetric image data 130a, 130b, and the temporal sequence of 2D angiographic images 140 into a neural network NN1; and
in response to the inputting, generating S140 the predicted temporal sequence of 3D angiographic images 110 representing the flow of the contrast agent through the region of interest 120; and
wherein the neural network NN1 is trained to predict the temporal sequence of 3D angiographic images 110 from the temporal sequence of 2D angiographic images 140, and to constrain the predicted temporal sequence of 3D angiographic images 110 by the volumetric image data 130a, 130b.

Since the predicted temporal sequence of 3D angiographic images is provided by a neural network from a temporal sequence of 2D angiographic images, the method provides the benefits of temporal sequences of 3D angiographic images, including structural information that enables a deeper understanding of the flow of a contrast agent in a region of interest, without the drawbacks of needing to accurately align an imaging system with the anatomy, and restricted access to the region of interest.

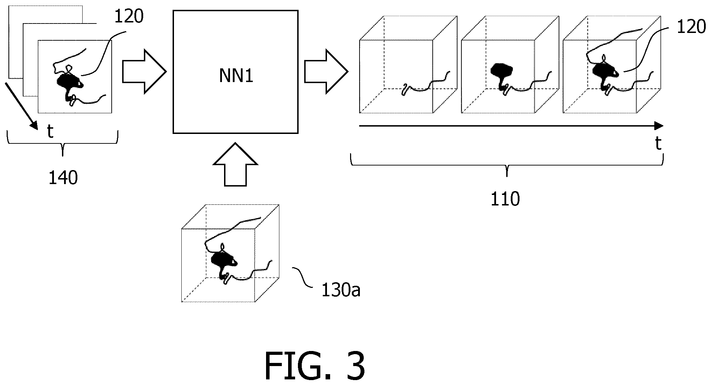

Fig. 2 is a schematic diagram illustrating an example of a system for providing a temporal sequence of 3D angiographic images 110 representing a flow of a contrast agent through a region of interest 120, in accordance with some aspects of the present disclosure. Operations described in relation to the method illustrated in Fig. 1, may also be performed by the system 200 illustrated in Fig. 2. Likewise, operations described in relation to the system 200 may also be performed in the method described with reference to Fig. 1. Fig. 3 is a schematic diagram illustrating a first example of a method of providing a temporal sequence of 3D angiographic images 110 representing a flow of a contrast agent through a region of interest 120, in accordance with some aspects of the present disclosure. With reference to Fig. 1 and Fig. 3, in the operation S110, volumetric image data representing the region of interest 120, is received. The region of interest may be an aneurism, for example, although the region of interest may in general be anywhere in the vasculature, as mentioned above.

The volumetric image data may in general be generated by a volumetric imaging system. A volumetric X-ray imaging system may be used, for example. A volumetric X-ray imaging system typically generates image data whilst rotating, or stepping, an X-ray source-detector arrangement around an imaging region, and subsequently reconstructs the image data obtained from multiple rotational angles into a 3D, or volumetric image. Examples of volumetric X-ray imaging systems include computed tomography "CT" imaging systems, cone beam CT "CBCT" imaging systems, and spectral CT imaging systems. In some examples, the volumetric image data may be generated using a digital subtraction angiography "DSA" technique. Alternatively, the volumetric image data may be generated by a magnetic resonance imaging "MRI" system. For example, magnetic resonance angiography "MRA" volumetric image data may be generated by injecting a contrast agent into the vasculature and using oscillating magnetic fields at specific resonance frequencies to generate images of various anatomical structures using an MRI imaging system.

In some examples, the volumetric image data may represent pre-procedural data generated prior to an insertion of an interventional device into the region of interest 120. The volumetric image data may be generated during a planning stage, or shortly before an interventional procedure, for example.

The volumetric image data received in the operation S110 may be received from a volumetric imaging system, or from a computer readable storage medium, or from the Internet or the Cloud, for example. The volumetric image data may be received by the one or more processors 170 illustrated in Fig. 2. The volumetric image data may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals.

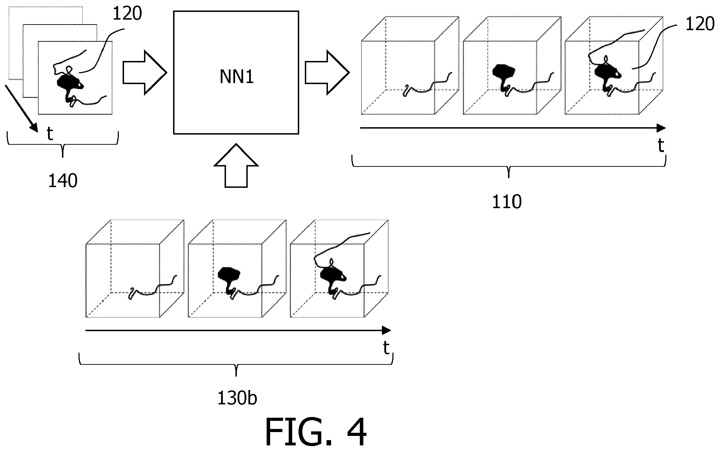

In one example, the volumetric image data includes a 3D angiographic image 130a representing the region of interest 120. This example is illustrated in Fig. 3. In this example, the 3D angiographic image 130a may be generated subsequent to the injection of a contrast agent. The contrast agent highlights the vasculature in the 3D angiographic image 130a. In another example, described later with reference to Fig. 4, the volumetric image data includes a temporal sequence of 3D angiographic images 130b representing a reference flow of the contrast agent through the region of interest 120. With reference to the Fig. 3 example, the volumetric image data may include a single 3D angiographic image 130a, i.e. one and only one 3D angiographic image 130a, or it may include multiple 3D angiographic images. The single 3D angiographic image 130a, may be generated by rotating a source-detector arrangement of a volumetric imaging system through a single complete rotation, or less, around the region of interest.

In the operation S120, a temporal sequence of 2D angiographic images 140 representing the flow of the contrast agent through the region of interest 120, is received. The temporal sequence of 2D angiographic images 140 may be generated by a projection X-ray imaging system that generates 2D X-ray images. Projection X-ray imaging systems typically include a support arm such as a so-called "C-arm", or an "O-arm", that supports an X-ray source-detector arrangement. Projection X-ray imaging systems may alternatively include a support arm with a different shape to these examples. Projection X-ray imaging systems typically generate projection X-ray images with the support arm held in a static position with respect to an imaging region during the acquisition of image data. In some examples, the temporal sequence of 2D angiographic images 140 may be generated using a digital subtraction angiography "DSA" technique. The X-ray projection images may be generated by the projection X-ray imaging system 180 illustrated in Fig. 2, for example. By way of an example, the angiographic images may be generated by the Philips Azurion 7 X-ray imaging system marketed by Philips Healthcare, Best, The Netherlands.

The temporal sequence of 2D angiographic images 140 that is received in the operation S120 may be received by the one or more processors 170 illustrated in Fig. 2. The temporal sequence of 2D angiographic images 140 may be received via any form of data communication, as described above for the volumetric image data. The temporal sequence of 2D angiographic images 140 represents the same region of interest as the volumetric image data 130a, 130b that is received in the operation S110.

In general, the temporal sequence of 2D angiographic images 140 that is received in the operation S 120 may be generated later in time than the volumetric image data 130a, 130b. The volumetric image data may represent pre-procedural data generated prior to an insertion of an interventional device into the region of interest 120, for example. The volumetric image data may be generated during a planning stage, or shortly before an interventional procedure, for example. The temporal sequence of 2D angiographic images 140 may represent intra-procedural data generated during an insertion of an interventional device into the region of interest 120, for example.

In the operation S130, the volumetric image data 130a, and the temporal sequence of 2D angiographic images 140, are inputted into a neural network NN1. This is illustrated in Fig. 3. In response to the inputting operation S130, in the operation S140 a predicted temporal sequence of 3D angiographic images 110 representing the flow of the contrast agent through the region of interest 120 is generated, as also illustrated in Fig. 3. The neural network NN1 is trained to predict the temporal sequence of 3D angiographic images 110 from the temporal sequence of 2D angiographic images 140, and to constrain the predicted temporal sequence of 3D angiographic images 110 by the volumetric image data 130a.

As mentioned above, in another example, the volumetric image data that is inputted in the operation S130, includes a temporal sequence of 3D angiographic images 130b representing a reference flow of the contrast agent through the region of interest 120. This example is described with reference to Fig. 4, which is a schematic diagram illustrating a second example of a method of providing a temporal sequence of 3D angiographic images 110 representing a flow of a contrast agent through a region of interest 120, in accordance with some aspects of the present disclosure. As in the Fig. 3 example, in the Fig. 4 example, the temporal sequence of 3D angiographic images 130b may be generated subsequent to the injection of a contrast agent. In the Fig. 4 example, the temporal sequence of 3D angiographic images represent a flow of the contrast agent. In general, the temporal sequence of 2D angiographic images 140 that is received in the operation S120 may be generated later in time than the volumetric image data 130a, 130b. The volumetric image data may represent pre-procedural data generated prior to an insertion of an interventional device into the region of interest 120, for example. The volumetric image data may be generated during a planning stage, or shortly before an interventional procedure, for example. The temporal sequence of 2D angiographic images 140 may represent intra-procedural data generated during an insertion of an interventional device into the region of interest 120, for example.

In the operation S130, the volumetric image data 130b, and the temporal sequence of 2D angiographic images 140, are inputted into a neural network NN1. This is illustrated in Fig. 4. In response to the inputting operation S130, in the operation S140 a predicted temporal sequence of 3D angiographic images 110 representing the flow of the contrast agent through the region of interest 120 is generated, as also illustrated in Fig. 4. The neural network NN1 is trained to predict the temporal sequence of 3D angiographic images 110 from the temporal sequence of 2D angiographic images 140, and to constrain the predicted temporal sequence of 3D angiographic images 110 by the volumetric image data 130b.

In the Fig. 3 example, and in the Fig. 4 example, the predicted temporal sequence of 3D angiographic images 110 is constrained by the volumetric image data 130a, and 130b respectively. This constraint may be applied by computing the value of a loss function representing a difference between the predicted temporal sequence of 3D angiographic images 110, and the volumetric image data 130a, 130b. At inference, the value of the loss function may be used to fine-tune the predictions of the neural network. This loss function has the effect of ensuring that the images in the predicted temporal sequence of 3D angiographic images 110 accurately depict the 3D structure of the region of interest in the volumetric image data 130a, 130b. Loss functions such as L1 or L2 loss, structural similarity index, Huber loss, log cosh loss, and so forth, may be used for this purpose. The value of this loss function may be used to fine-tune the predictions of the neural network by comparing the 3D structure of the region of interested in the predicted temporal sequence of 3D angiographic images 110 and the 3D structure of the region of interested in the volumetric image data 130a, 130b. This comparison may, for instance, be performed by computing a composite or trace 3D angiographic image from the predicted temporal sequence of 3D angiographic images 110. The composite image may represent the maximum value or the minimum value or the average value of the image intensity values in the temporal sequence of 3D angiographic images 110. The composite image captures the full 3D structure of the vasculature through which contrast flow was captured in the temporal sequence of 3D angiographic images 110. Therefore, a structural constraint on the predicted temporal sequence of 3D angiographic images 110 may be applied by computing the value of a first loss function representing a difference between the composite 3D angiographic image computed from the predicted temporal sequence of 3D angiographic images 110 and the inputted single 3D angiographic image 130a or the composite 3D angiographic image generated from the inputted temporal sequence of 3D angiographic images 130b. Alternatively or additionally, a flow constraint may be applied by computing, the value of a second loss function representing a difference between synthetic projection images generated by projecting the predicted 3D angiographic images 110, onto a virtual detector plane corresponding to a detector plane of the inputted 2D angiographic images 140, and the inputted 2D angiographic images 140. At inference, the value of the second loss function may likewise be used to fine-tune the predictions of the neural network. This second loss function has the effect of ensuring that the flow in the images in the predicted temporal sequence of 3D angiographic images 110 accurately depicts the flow of contrast agent in the inputted 2D angiographic images 140. When two loss functions are used to apply this constraint, the values of the first and second loss functions may be weighted and summed to provide a combined loss value. At inference, the combined loss value may be used to fine-tune the predicted temporal sequence of 3D angiographic images 110 as described above.

The virtual detector plane may be determined by identifying the detector plane of the inputted 2D angiographic images 140 using position encoder data that represents an orientation of the X-ray imaging system that generates the 2D angiographic images 140, respective the region of interest in a patient. Alternatively, the virtual detector plane may be determined by registering the inputted 2D angiographic images 140 to the inputted volumetric image data 130a, 130b and determining the orientation of the X-ray imaging system from which the inputted 2D angiographic images 140 were generated based on the registration.

In some examples, confidence values for the predicted temporal sequence of 3D angiographic images 110, may also be generated. Outputting the confidence values permits a user to modulate their trust in the predictions of the neural network NN1. The confidence values may be calculated using various techniques. In one example, the values of the loss functions that are generated by the neural network NN1 as described above are used to provide the confidence values. In this example, a relatively lower value of the loss function results in a relatively higher confidence value, and vice versa. In another example, confidence values may be assigned to the predictions of the neural network NN1 by observing attention maps produced by the network and evaluating whether the attention of the network was within the region of interest, in which case the confidence value may be relatively higher, or elsewhere, in which case the confidence value may be relatively lower.

In another example, confidence values for the predicted temporal sequence of 3D angiographic images 110 are generated based on a count of the total number of 2D angiographic images that are used to generate each predicted 3D angiographic image. In this example, the confidence value for an image may be relatively lower if a single 2D angiographic image is used to generate the predicted 3D angiographic image, whereas the confidence value may be relatively higher if multiple 2D angiographic images are used.

In another example, confidence values for the predicted temporal sequence of 3D angiographic images 110 are generated by calculating one or more analytical metrics such as a degree of overlap, or a degree of foreshortening, for features in the region of interest. A technique for calculating such analytical metrics, is disclosed in a document by Wilson, D., et al., entitled "Determining X-ray projections for coil treatments of intracranial aneurysms" in IEEE Transactions on Medical Imaging, vol. 18, no. 10, pp. 973-980, Oct. 1999, doi: 10.1109/42.811309. In this document, an algorithm is presented for computing factors such as the aforementioned degree of overlap, and degree of foreshortening. The algorithm uses as inputs i) a discretely defined 3D segmentation of a region of interest and surrounding anatomy, and ii) a model of the geometric and imaging parameters of an X-ray imaging system. In this example, values for the analytical metrics may be calculated for the inputted temporal sequence of 2D angiographic images 140, based on the volumetric image data 130a, 130b, which provides a model of the region of interest, and these values may be used to generate the confidence values for the predicted temporal sequence of 3D angiographic images 110. In this example, a high degree of overlap in an inputted 2D angiographic image 140, may be indicative of a relatively lower confidence in the output of the neural network NN1, and vice versa.

In another example, the dropout technique may be used to provide confidence values for the predicted temporal sequence of 3D angiographic images 110. The dropout technique involves iteratively inputting the same data into a neural network and determining the neural network's output whilst randomly excluding a proportion of the neurons from the neural network in each iteration. The outputs of the neural network are then analyzed to provide mean and variance values. The mean value represents the final output, and the magnitude of the variance indicates whether the neural network is consistent in its predictions, in which case the variance is small and the confidence value is relatively higher, or whether the neural network was inconsistent in its predictions, in which case the variance is larger and the confidence value is relatively lower.

Thus, confidence values for the predicted temporal sequence of 3D angiographic images 110 may be computed by one or more of:
determining a value of a first loss function LF₁ representing a difference between the received volumetric image data 130a, 130b and the predicted 3D angiographic images 110;
determining a value of a second loss function LF₂ representing a difference between synthetic projection images generated by projecting the predicted 3D angiographic images 110, onto a virtual detector plane corresponding to a detector plane of the inputted 2D angiographic images 140, and the inputted 2D angiographic images 140;
generating an attention map indicating a location of one or more factors on which the predicted 3D angiographic images 110, are based, and determining a difference between the attention map and a location of the region of interest 120; and
computing a total number of the temporal sequences of 2D angiographic training images used to generate the predicted 3D angiographic images 110.

The neural network NN1 may include one or more architectures, such as a convolutional neural network "CNN", a recurrent neural network "RNN", a temporal convolutional network "TCN", an encoder-decoder, a variational encoder-decoder, a transformer, and a generative adversarial network "GAN", for example. The neural network NN1 may include multiple fully connected or convolutional layers with pooling, batch normalization, dropout, non-linear operations, etc. between them. The neural network may generate the predicted temporal sequence of 3D angiographic images 110 with the same spatial dimensions as the inputted volumetric image data, and same the temporal resolution as the inputted temporal sequence of 2D angiographic images 140. For example, the neural network NN1 may output an n×m×p×t output of grey levels where n×m×p are the dimensions of the volumetric image data and t is the number of frames in the inputted temporal sequence of 2D angiographic images 140. The grey levels in the output image indicate the updated contrast flow patterns based on the flow observed intraoperatively in the temporal sequence of 2D angiographic images 140.

Detail on training the neural network NN1 is provided below.

Thus, in both the Fig. 3 example and the Fig. 4 example, the predicted temporal sequence of 3D angiographic images 110 is generated from the inputted temporal sequence of 2D angiographic images 140. As mentioned above, the temporal sequence of 2D angiographic images 140 may be generated later in time than the volumetric image data 130a, 130b. In so doing, the method enables a clinical investigation to take place using a temporal sequence of 2D angiographic images 140, and provides the benefits of a temporal sequence of 3D angiographic images, without the need to concurrently perform a 3D angiographic imaging operation on the region of interest. The volumetric image data may for example represent pre-procedural data generated prior to an insertion of an interventional device into the region of interest 120, and the temporal sequence of 2D angiographic images 140 may for example represent intra-procedural data generated during an insertion of an interventional device into the region of interest 120. Consequently, an interventional procedure may be performed with the interventional device without the need to acquire an intra-procedural temporal-sequence of 3D angiographic images.

The predicted temporal sequence of 3D angiographic images 110 may be used for various purposes. For example, the 3D angiographic images 110 may be outputted to a display, such as the display 190 illustrated in Fig. 2. A user input device, such as a touchscreen, a mouse and so forth, may also be provided in order to enable a user to adjust a viewing angle in order to view the 3D angiographic images 110 from a desired orientation with respect to the region of interest. In one example, differential images may also be generated and outputted. The differential images represent changes in the flow of the contrast agent through the region of interest 120. The differential images may represent the change in contrast flow between consecutive angiographic images, or between angiographic images acquired at the beginning of the procedure and angiographic images acquired in the middle of the procedure, for example.

In one example, the predicted 3D angiographic images are projected in order to provide 2D angiographic images. In this example, the method described above with reference to Fig. 1 may also include:
projecting the predicted temporal sequence of 3D angiographic images 110 onto a virtual plane of an X-ray detector 150a of an X-ray imaging system 150 to provide a predicted temporal sequence of synthetic 2D angiographic images representing the flow of the contrast agent through the region of interest.

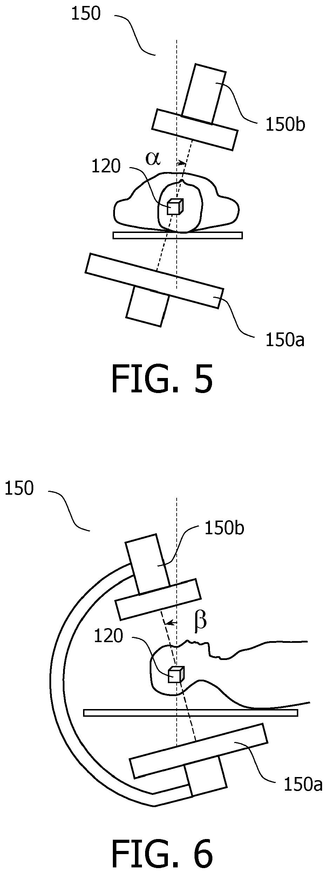

In this example, the synthetic 2D angiographic images may be generated by projecting the predicted 3D angiographic images from any orientation with respect to the region of interest. Synthetic 2D angiographic images may be generated from multiple orientations with respect to the region of interest in order to facilitate a deeper understanding of the flow of the contrast agent in the region of interest, and to better visualize the region of interest without having to position the X-ray imaging system in multiple configurations and exposing the patient and staff to X-ray radiation due to multiple image acquisitions. The synthetic 2D angiographic images may be obtained using the predicted 3D angiographic images and a model of the geometry of the X-ray imaging system. The model may for example include parameters such as the relative positions of an X-ray source, an X-ray detector, and a region of interest, and the dimensions of the X-ray detector. The projections may then be obtained by adjusting an orientation a, b of the X-ray source 150b and the X-ray detector 150a with respect to the region of interest 120, and projecting the 3D angiographic images onto the detector. Fig. 5 is a schematic diagram illustrating an orientation of an X-ray source 150b and an X-ray detector 150a of an X-ray imaging system 150 respective a region of interest 120 in a patient, including a rotational angle a of a central ray of the projection X-ray imaging system 150 around a longitudinal axis of a patient, in accordance with some aspects of the present disclosure. Fig. 6 is a schematic diagram illustrating an orientation of an X-ray source 150b and an X-ray detector 150a of an X-ray imaging system 150 respective a region of interest 120 in a patient, including a tilt angle b of a central ray of the projection X-ray imaging system 150 with respect to a cranial-caudal axis of a patient, in accordance with some aspects of the present disclosure. The rotational angle a and the tilt angle b may be adjusted with respect to the region of interest 120, and used to obtain the desired synthetic 2D angiographic images.

In one example, the synthetic 2D angiographic images that are provided in this manner are used to provide a different view of the region of interest to the view provided by the temporal sequence of 2D angiographic images 140 that are inputted into the neural network NN1. In this example, the projecting operation described above may also include projecting the predicted temporal sequence of 3D angiographic images 110 onto the virtual plane of the X-ray detector 150a of the X-ray imaging system 150 at an orientation a, b of an X-ray source 150b and the X-ray detector 150a of the X-ray imaging system 150 with respect to the region of interest 120, such that the predicted temporal sequence of synthetic 2D angiographic images represents a different view of the region of interest to the received temporal sequence of 2D angiographic images.

In one example, the neural network NN1 is trained to predict the temporal sequence of 3D angiographic images 110 from two temporal sequences of 2D angiographic images representing different views of the flow of the contrast agent through the region of interest 120. Temporal sequences of 2D angiographic images that have different views of the flow of the contrast agent through a region of interest may be obtained using an angiographic imaging system that has multiple X-ray source-detector arrangements, such as a biplane X-ray imaging system. In such imaging systems, the orientation of each source-detector arrangement with respect to the region of interest, is different. The orientations may be arranged orthogonally to one another, for example, or they may be arranged at another angle to one another. Different views may be used to provide additional detail on a region of interest. In this example, the information from multiple views is used to improve the predicted temporal sequence of 3D angiographic images 110 generated by the neural network. The information from multiple views may improve the prediction of the 3D structure of the region of interest. The information from multiple views may also assist the neural network to resolve ambiguity in images in which a flow of contrast agent appears to slow-down by explaining the slow-down as the result of a change in vasculature diameter, or the result of foreshortening.

In this example, the method described with reference to Fig. 1 also includes:
receiving a second temporal sequence of 2D angiographic images representing the flow of the contrast agent through the region of interest 120, the second temporal sequence of 2D angiographic images representing a different view of the region of interest 120 to the temporal sequence of 2D angiographic images 140;
inputting the second temporal sequence of 2D angiographic images into the neural network NN1; and
in response to the inputting, generating the predicted temporal sequence of 3D angiographic images 110 based further on the second temporal sequence of 2D angiographic images; and
wherein the neural network NN1 is trained to predict the temporal sequence of 3D angiographic images 110 from two temporal sequences of 2D angiographic images representing different views of the flow of the contrast agent through the region of interest 120, and to constrain the predicted temporal sequence of 3D angiographic images 110 by the volumetric image data 130a, 130b.

In one example, the predicted temporal sequence of 3D angiographic images 110 is generated for a specific region of the inputted 2D angiographic images. In this example, the method described with reference to Fig. 1 also includes receiving user input indicating an extent of the region of interest in the volumetric image data 130a, 130b and/or in the 2D angiographic images 140; and outputting the predicted temporal sequence of 3D angiographic images 110 corresponding to the indicated extent of the region of interest in the volumetric image data 130a, 130b and/or in the 2D angiographic images 140. The user input may be provided by a user in the form of annotations on the 2D angiographic images 140, for example. A user may manually draw a bounding box around a region of interest, for example. The region of interest may also be identified automatically, following which a user selects the automatically-identified region of interest. Techniques for the automatic identification of a region of interest include image intensity thresholding, region growing, template matching, level sets, active contour modelling, segmentation with a neural network (e.g., U-Nets), feature detection, and so forth. The inference time of the neural network NN1 may be reduced by indicating the extent of the region of interest in the volumetric image data and/or in the 2D angiographic images that are inputted into the neural network NN1.

In another example, the predicted temporal sequence of 3D angiographic images 110 is generated with a user-specified temporal resolution, or a user-specified temporal window. In these examples, the method described with reference to Fig. 1 also includes receiving user input indicating a temporal resolution for the predicted temporal sequence of 3D angiographic images 110; and/or
receiving user input indicating a temporal window for the predicted temporal sequence of 3D angiographic images 110; and
outputting the predicted temporal sequence of 3D angiographic images 110 with the indicated temporal resolution and/or within the indicated temporal window, respectively.

User input indicative of the temporal resolution, or the temporal window, may be provided by a user input device such as a mouse, a touchscreen, and so forth, in combination with a graphical user interface "GUI". The GUI may for example include a time interval slider that allows a user to choose frames in the predicted temporal sequence of 3D angiographic images 110. The predicted temporal sequence of 3D angiographic images 110 may be color-coded by using different colors to represent the time of arrival of the contrast agent at different locations in the vasculature. The GUI may also include a window size slider that allows the user to select the temporal resolution of the predicted temporal sequence of 3D angiographic images 110. The GUI may permit the display of synthetic projections of the temporal sequence of 3D angiographic images 110 from a user-defined orientation respective the region of interest.

As mentioned above, in some examples, the temporal sequence of 2D angiographic images 140 may be generated later in time than the volumetric image data 130a, 130b. For example, the volumetric image data 130a, 130b may represent pre-procedural data generated prior to an insertion of an interventional device into the region of interest 120, and the received temporal sequence of 2D angiographic images 140 may represent intra-procedural data generated during an insertion of the interventional device into the region of interest 120. Thus, in one example, the flow of the contrast agent through the region of interest 120 in the volumetric image data 130a, 130b is different to the flow of the contrast agent through the region of interest 120 in the received temporal sequence of 2D angiographic images 140.

In this example, the neural network NN1 may also be trained to predict a proportion of an interventional procedure on the region of interest that has been completed. The neural network NN1 may be trained to do this by including in the training data, 3D angiographic training images, and a corresponding temporal sequence of 2D angiographic training images, the latter representing a flow of a contrast agent through the region of interest during interventional procedures on a region of interest, and training the neural network NN1 to predict a ground truth proportion of an interventional procedure on the region of interest that has been completed. The ground truth proportion of the interventional procedure on the region of interest that has been completed, may be calculated from historic procedures. During training, the ground truth, and predicted proportion of the interventional procedure on the region of interest that has been completed, can be compared using a loss function, the value of which contributes to the value of the loss function that is used to train the neural network NN1.At inference, the neural network NN1 then predicts the proportion of the interventional procedure on the region of interest that has been completed, based on the flow in the predicted temporal sequence of 3D angiographic images. In this example, the neural network NN1 is trained to predict, for the images in the inputted temporal sequence of 2D angiographic images 140, a proportion of an interventional procedure on the region of interest that has been completed using the interventional device, and the method includes:
outputting the proportion of the interventional procedure on the region of interest that has been completed for the predicted temporal sequence of 3D angiographic images 110.

The training of the neural network NN1 is described below.

In general, the training of a neural network involves inputting a training dataset into the neural network, and iteratively adjusting the neural network's parameters until the trained neural network provides an accurate output. Training is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The process of training the neural network NN1 described above therefore includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean squared error, or the Huber loss, or the cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

In one example, the neural network NN1 is trained to predict the temporal sequence of 3D angiographic images 110 from the temporal sequence of 2D angiographic images 140, and to constrain the predicted temporal sequence of 3D angiographic images by the volumetric image data 130a; by
receiving S210, for each of a plurality of patients, a 3D angiographic training image 130'a representing the region of interest 120;
receiving S220, for each patient, a temporal sequence of 2D angiographic training images 140' corresponding to the 3D angiographic training image 130'a, the 2D angiographic training images 140' representing a flow of a contrast agent through the region of interest 120; and
for each of a plurality of 2D angiographic training images 140' in a temporal sequence for a patient, and for each of a plurality of patients:
   inputting S230 the 2D angiographic training image 140' into the neural network NN1;
   generating S240 a corresponding predicted 3D angiographic image 110' representing the flow of the contrast agent through the region of interest 120;
   adjusting S250 parameters of the neural network NN1 based on:
      i) a value of a first loss function LF₁ representing a difference between the received 3D angiographic training image 130'a and the predicted 3D angiographic image 110'; and
      ii) a value of a second loss function LF₂ representing a difference between the inputted 2D angiographic training image 140' and a projection 160 of the predicted 3D angiographic image 110' onto a plane corresponding to a plane of the inputted 2D angiographic training image 140'; and repeating S260 the inputting S230 and the generating S240 and the adjusting S250 until a stopping criterion is met.

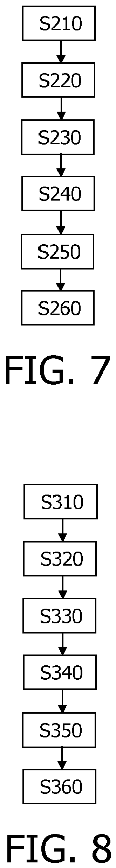

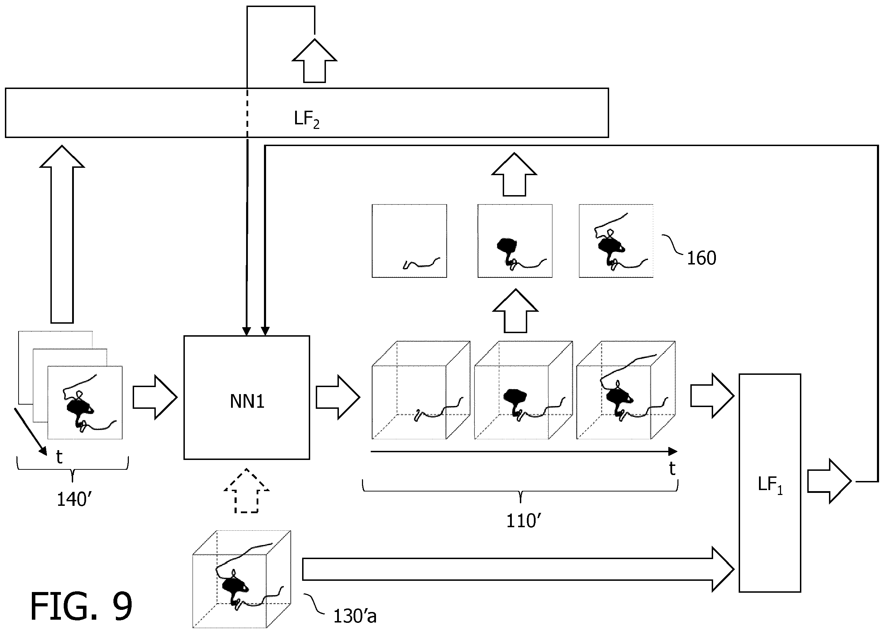

This training method is illustrated in Fig. 7, which is a flowchart illustrating a first example of a method of training a neural network to provide a temporal sequence of 3D angiographic images representing a flow of a contrast agent through a region of interest, in accordance with some aspects of the present disclosure. The training method is also illustrated in Fig. 9, which is a schematic diagram illustrating a first example of a method of training a neural network to provide a temporal sequence of 3D angiographic images representing a flow of a contrast agent through a region of interest, in accordance with some aspects of the present disclosure.

With reference to Fig. 7, and Fig. 9, in the operation S210, a 3D angiographic training image 130'a representing the region of interest 120, is received for a plurality of patients. The 3D angiographic training images may be generated by a 3D angiographic imaging system, such as a computed tomography angiographic "CTA", 3D rotational angiographic "3DRA", or a magnetic resonance angiographic "MRA" imaging system, for example. Three-dimensional angiographic training images 130'a for one hundred or more patients may be received in the operation S210. The images may include a range of various abnormalities for a region of interest. The images may represent patients having different ages, genders, body mass index, and so forth. The 3D angiographic training images 130'a may be generated prior to an interventional procedure on the region of interest.

In the operation S220, a temporal sequence of 2D angiographic training images 140' is received for each of a plurality of patients. The 2D angiographic training images 140' represent a flow of a contrast agent through the region of interest 120. The 2D angiographic training images 140' may for example represent the flow of a contrast agent during an interventional procedure on the region of interest. The 2D angiographic training images 140' may be generated by a projection X-ray imaging system. Alternatively, the 2D angiographic training images 140' may be synthetic 2D angiographic images that are generated by projecting the 3D angiographic images generated by a volumetric X-ray imaging system onto a virtual detector plane, as described above.

In the operation S230, multiple 2D angiographic training images 140' from the temporal sequences of multiple patients are inputted into the neural network NN1. In the operation S240, the neural network NN1 makes predictions; and in the operation S250 the parameters of the neural network NN1 are adjusted based on the values of two loss functions, LF₁ and LF₂. The temporal sequences may be selected at random from the plurality of patients in the training dataset prior to inputting these into the neural network NN 1. By changing the order of inputs in this manner may improve predictions by preventing the neural network from memorizing an order of predictions for a set order of inputs. In the operation S260, the operations S230, S240 and S250 are repeated multiple times. The calculation of these loss functions is illustrated schematically in Fig. 9. The value of the first loss function LF₁ represents a difference between the received 3D angiographic training image 130'a and the predicted 3D angiographic image 110'. The value of the second loss function LF₂ represents a difference between the inputted 2D angiographic training image 140' and a projection 160 of the predicted 3D angiographic image 110' onto a plane corresponding to a plane of the inputted 2D angiographic training image 140'. The plane onto which the predicted 3D angiographic image 110' is projected, may be determined as described above, i.e. it may be known based on position encoder data representing an orientation of the X-ray imaging system that generates the 2D angiographic training images 140', respective the region of interest in a patient. Alternatively, the plane onto which the predicted 3D angiographic image 110' is projected, may be determined by registering the 2D angiographic training images 140' to the 3D angiographic training image 130'a. The values of the loss functions may be calculated using loss function such as L1 or L2 loss, structural similarity index, Huber loss, log cosh loss, and so forth. The values of the loss functions may be weighted and summed to provide a combined value. The backpropagation technique described above may be used to adjust the parameters of the neural network NN1 using the combined value for the loss functions LF₁ and LF₂.

The values of the first and second loss functions, i.e. LF₁ and LF₂, that are used during training, are calculated in the same manner as the first and second loss functions described in relation to Fig. 3 and Fig. 4. The loss functions also have similar effects to those described above. The first loss function LF₁ has the effect of ensuring that the images in the predicted temporal sequence of 3D angiographic images 110 accurately depict the 3D structure of the region of interest in the 3D angiographic training image 130'a. The second loss function LF₂ has the effect of ensuring that the flow in the images in the predicted temporal sequence of 3D angiographic images 110' accurately depicts the flow of contrast agent in the inputted 2D angiographic training images 140'.

In another example, the neural network NN1 is trained to predict the temporal sequence of 3D angiographic images 110 from the temporal sequence of 2D angiographic images 140, and to constrain the predicted temporal sequence of 3D angiographic images by the volumetric image data 130b; by
receiving S310, for each of a plurality of patients, a temporal sequence of 3D angiographic training images 130'b representing a reference flow of the contrast agent through the region of interest 120;
receiving S320, for each patient, a temporal sequence of 2D angiographic training images 140' corresponding to the temporal sequence of 3D angiographic training images 130b', the temporal sequence of 2D angiographic training images 140' representing a flow of a contrast agent through the region of interest 120; and
for each of a plurality of 2D angiographic training images 140' in a temporal sequence for a patient, and for each of a plurality of patients:
   inputting S330 the 2D angiographic training image 140' into the neural network NN1;
   generating S340 a corresponding predicted 3D angiographic image 110' representing the flow of the contrast agent through the region of interest 120;
   adjusting S350 parameters of the neural network NN1 based on:
      i) a value of a first loss function LF₁ representing a difference between a corresponding 3D angiographic training image from the received temporal sequence of 3D angiographic images 140'b and the predicted 3D angiographic images 110'; and
      ii) a value of a second loss function LF₂ representing a difference between the inputted 2D angiographic training image 140' and a projection 160 of the predicted 3D angiographic image 110' onto a plane corresponding to a plane of the inputted 2D angiographic training image 140'; and
      repeating S360 the inputting S330 and the generating S340 and the adjusting S350 until a stopping criterion is met.

This training method is illustrated in Fig. 8, which is a flowchart illustrating a second example of a method of training a neural network to provide a temporal sequence of 3D angiographic images representing a flow of a contrast agent through a region of interest, in accordance with some aspects of the present disclosure. In this second example, the training the neural network NN1 is performed in the same manner as described above for the first example, with the difference that rather than performing training with a single 3D angiographic training image 130'a representing the region of interest 120, a temporal sequence of 3D angiographic training images 130'b, is used. Also, when the value of the first loss function is calculated, in the second example, the value of the first loss function LF₁ represents a difference between a corresponding 3D angiographic training image from the received temporal sequence of 3D angiographic images 140'b and the predicted 3D angiographic image 110'. The images correspond in the sense that they represent the same point in time. Thus, in this example, the predicted 3D angiographic images 110' may have the same time interval as the temporal sequence of 3D angiographic training images 130'b.

In the training method described with reference to Fig. 7, or in the training method described with reference to Fig. 8, further operations may also be performed. For example, user input indicating an extent of the region of interest 120 in the temporal sequences of 3D angiographic training images 130'b and/or 2D angiographic training images 140' may be received, and the value of the first loss function LF₁ and/or the second loss function LF₂ may be computed only within the indicated extent. In so doing, the training is tailored to the region of interest. Alternatively or additionally, the value of the first loss function LF₁ and/or the second loss function LF₂ may be computed by applying a higher weighting to the respective loss function within the indicated extent, as compared to outside the indicated extent. This also has the effect of tailoring the training to the region of interest.

In the training method described with reference to Fig. 7, or in the training method described with reference to Fig. 8, the training of the neural network NN1 may also include inputting the 3D angiographic training image 130'a, or the temporal sequence of 3D angiographic training images 130'b, into the neural network NN1. This is illustrated in Fig. 9 by way of the dashed arrow between these items. Inputting the 3D angiographic training image(s) in to the neural network NN1 in this manner may simplify training of the neural network since it only needs to estimate changes in 3D shape or flow in the region of interest based on the flow of the contrast agent in the inputted 2D angiographic training images 140'. By contrast, if the 3D angiographic training image(s) are not inputted into the neural network NN1, the neural network NN1 learns to predict the structural shape, and the flow of the contrast agent, from the inputted 2D angiographic training images 140', and the 3D angiographic training image(s) are simply used to calculate the value of the second loss function LF₁.

In another example, in the training method described with reference to Fig. 7, or in the training method described with reference to Fig. 8, the 3D angiographic training image 130'a or the temporal sequence of 3D angiographic training images 130'b, may represent pre-procedural data generated prior to an insertion of an interventional device into the region of interest. The temporal sequence of 2D angiographic training images 140' may represent intra-procedural data generated during an insertion of the interventional device into the region of interest. Moreover, the flow of the contrast agent through the region of interest 120' in the 3D angiographic training image 130'a, or in the temporal sequence of 3D angiographic training images 130'b may be different to the flow of the contrast agent through the region of interest in the temporal sequence of 2D angiographic training images 140'.

In another example, the training method described with reference to Fig. 8, may also include:
receiving a temporal sequence of 3D angiographic training images representing a flow of the contrast agent through the region of interest 120 during or after an insertion of the interventional device into the region of interest; and
   wherein the adjusting S250, S350 parameters of the neural network NN1, is based further on:
iii) a value of a third loss function LF₃ representing a difference between a corresponding 3D angiographic training image representing a flow of the contrast agent through the region of interest during or after an insertion of the interventional device into the region of interest, and the predicted 3D angiographic image 110'.

In this example, training data in the form of temporal sequences of intra-operative and/or post-operative 3D angiographic images of the region of interest are additionally available. The value of the third loss function LF₃ may be calculated in the same manner as the first and second loss functions LF₁ and LF₂ described above. In this example, these temporal sequences are used during training to adjust the parameters of the neural network NN1.Using the third loss function in this manner may provide for faster training of the neural network NN1 since it provides direct information to the neural network NN1 about the intraoperative and/or postoperative 3D structure of the region of interest.

In one example, the neural network NN1 that is trained is a GAN. The GAN may further use adversarial loss and/or cycle consistency loss to ensure the predicted temporal sequence of 3D angiographic images are in the "style" of the 3D angiographic training image(s). At inference, contrastive loss may be used to ensure that corresponding frames of the inputted temporal sequence of 2D angiographic images 140 are aligned with projections of the predicted 3D angiographic image 110' onto a plane corresponding to a plane of the inputted 2D angiographic training image 140'.

In another example, a computer program product is provided. The computer program product includes instructions which when executed by one or more processors, cause the one or more processors to carry out a method of providing a temporal sequence of 3D angiographic images 110 representing a flow of a contrast agent through a region of interest 120, comprising:
receiving S110 volumetric image data 130a, 130b representing the region of interest 120;
receiving S120 a temporal sequence of 2D angiographic images 140 representing the flow of the contrast agent through the region of interest 120;
inputting S130 the volumetric image data 130a, 130b, and the temporal sequence of 2D angiographic images 140 into a neural network NN1; and
in response to the inputting, generating S140 the predicted temporal sequence of 3D angiographic images 110 representing the flow of the contrast agent through the region of interest 120; and
wherein the neural network NN1 is trained to predict the temporal sequence of 3D angiographic images 110 from the temporal sequence of 2D angiographic images 140, and to constrain the predicted temporal sequence of 3D angiographic images 110 by the volumetric image data 130a, 130b.

As mentioned above, features of the computer implemented method, may also be implemented in a system. Thus, in one example, a system 200 for providing a temporal sequence of 3D angiographic images 110 representing a flow of a contrast agent through a region of interest 120, is provided. The system 200 includes one or more processors 170 configured to:
receive S110 volumetric image data 130a, 130b representing the region of interest 120;
receive S120 a temporal sequence of 2D angiographic images 140 representing the flow of the contrast agent through the region of interest 120;
input S130 the volumetric image data 130a, 130b, and the temporal sequence of 2D angiographic images 140 into a neural network NN1; and
in response to the input, generate S140 the predicted temporal sequence of 3D angiographic images 110 representing the flow of the contrast agent through the region of interest 120; and
wherein the neural network NN1 is trained to predict the temporal sequence of 3D angiographic images 110 from the temporal sequence of 2D angiographic images 140, and to constrain the predicted temporal sequence of 3D angiographic images 110 by the volumetric image data 130a, 130b.

An example of this system 200 is illustrated in Fig. 2. The system 200 includes one or more processors 170 that may perform the operations described above with reference to the flowchart in Fig. 1. The system 200 may also include one or more of: a projection X-ray imaging system 180 for generating the temporal sequences of 2D angiographic images 140; a display 190 for displaying the predicted temporal sequence of 3D angiographic images 110; a GUI; and a user input device configured to receive user input (not illustrated in Fig. 1), such as a keyboard, a mouse, a touchscreen, and so forth.

The above examples are to be understood as illustrative of the present disclosure, and not restrictive. Further examples are also contemplated. For instance, the examples described in relation to computer-implemented methods, may also be provided by the computer program product, or by the computer-readable storage medium, or by the system 200, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of providing a temporal sequence of 3D angiographic images (110) representing a flow of a contrast agent through a region of interest (120), the method comprising:
receiving (S110) volumetric image data (130a, 130b) representing the region of interest (120);
receiving (S120) a temporal sequence of 2D angiographic images (140) representing the flow of the contrast agent through the region of interest (120);
inputting (S130) the volumetric image data (130a, 130b), and the temporal sequence of 2D angiographic images (140) into a neural network (NN1); and
in response to the inputting, generating (S140) the predicted temporal sequence of 3D angiographic images (110) representing the flow of the contrast agent through the region of interest (120); and
wherein the neural network (NN1) is trained to predict the temporal sequence of 3D angiographic images (110) from the temporal sequence of 2D angiographic images (140), and to constrain the predicted temporal sequence of 3D angiographic images (110) by the volumetric image data (130a, 130b).

2. The computer-implemented method according to claim 1, wherein the volumetric image data (130a, 130b) comprises:
a 3D angiographic image (130a) representing the region of interest (120); or
a temporal sequence of 3D angiographic images (130b) representing a reference flow of the contrast agent through the region of interest (120).

3. The computer-implemented method according to claim 1 or claim 2, further comprising:
projecting the predicted temporal sequence of 3D angiographic images (110) onto a virtual plane of an X-ray detector (150a) of an X-ray imaging system (150) to provide a predicted temporal sequence of synthetic 2D angiographic images representing the flow of the contrast agent through the region of interest.

4. The computer-implemented method according to claim 3, wherein the projecting comprises projecting the predicted temporal sequence of 3D angiographic images (110) onto the virtual plane of the X-ray detector (150a) of the X-ray imaging system (150) at an orientation (a, b) of an X-ray source (150b) and the X-ray detector (150a) of the X-ray imaging system (150) with respect to the region of interest (120), such that the predicted temporal sequence of synthetic 2D angiographic images represents a different view of the region of interest to the received temporal sequence of 2D angiographic images.

5. The computer-implemented method according to claim 1, further comprising:
receiving a second temporal sequence of 2D angiographic images representing the flow of the contrast agent through the region of interest (120), the second temporal sequence of 2D angiographic images representing a different view of the region of interest (120) to the temporal sequence of 2D angiographic images (140);
inputting the second temporal sequence of 2D angiographic images into the neural network (NN1); and
in response to the inputting, generating the predicted temporal sequence of 3D angiographic images (110) based further on the second temporal sequence of 2D angiographic images; and
wherein the neural network (NN1) is trained to predict the temporal sequence of 3D angiographic images (110) from two temporal sequences of 2D angiographic images representing different views of the flow of the contrast agent through the region of interest (120), and to constrain the predicted temporal sequence of 3D angiographic images (110) by the volumetric image data (130a, 130b).

6. The computer-implemented method according to claim 1, further comprising; computing confidence values for the predicted temporal sequence of 3D angiographic images (110).

7. The computer-implemented method according to claim 6, wherein the confidence values are computed by:
determining a value of a first loss function (LF₁) representing a difference between the received volumetric image data (130a, 130b) and the predicted 3D angiographic images (110); and/or
determining a value of a second loss function (LF₂) representing a difference between synthetic projection images generated by projecting the predicted 3D angiographic images (110), onto a virtual detector plane corresponding to a detector plane of the inputted 2D angiographic images (140), and the inputted 2D angiographic images (140); and/or
generating an attention map indicating a location of one or more factors on which the predicted 3D angiographic images (110), are based, and determining a difference between the attention map and a location of the region of interest (120); and/or
computing a total number of the temporal sequences of 2D angiographic training images used to generate the predicted 3D angiographic images (110).

8. The computer-implemented method according to any previous claim, further comprising:
receiving user input indicating an extent of the region of interest in the 3D angiographic images (130a, 130b); and/or
receiving user input indicating an extent of the region of interest in the 2D angiographic images (140); and/or
receiving user input indicating a temporal resolution for the predicted temporal sequence of 3D angiographic images (110); and/or
receiving user input indicating a temporal window for the predicted temporal sequence of 3D angiographic images (110); and
outputting the predicted temporal sequence of 3D angiographic images (110) corresponding to the indicated extent of the region of interest in the 3D angiographic images (130a, 130b) and/or corresponding to the indicated extent of the region of interest in the 2D angiographic images (140) and/or with the indicated temporal resolution and/or within the indicated temporal window, respectively.

9. The computer-implemented method according to any previous claim, wherein the volumetric image data (130a, 130b) represents pre-procedural data generated prior to an insertion of an interventional device into the region of interest (120), and wherein the received temporal sequence of 2D angiographic images (140) represent intra-procedural data generated during an insertion of the interventional device into the region of interest (120), and wherein a flow of a contrast agent through the region of interest (120) in the volumetric image data (130a, 130b) is different to the flow of the contrast agent through the region of interest (120) in the received temporal sequence of 2D angiographic images (140).

10. The computer-implemented method according to claim 9, wherein the neural network (NN1) is trained to predict, for the images in the inputted temporal sequence of 2D angiographic images (140), a proportion of an interventional procedure on the region of interest that has been completed using the interventional device, and further comprising:
outputting the proportion of the interventional procedure on the region of interest that has been completed for the predicted temporal sequence of 3D angiographic images (110).

11. The computer-implemented method according to claim 1, wherein the neural network (NN1) is trained to predict the temporal sequence of 3D angiographic images (110) from the temporal sequence of 2D angiographic images (140), and to constrain the predicted temporal sequence of 3D angiographic images by the volumetric image data (130a); by
receiving (S210), for each of a plurality of patients, a 3D angiographic training image (130'a) representing the region of interest (120);
receiving (S220), for each patient, a temporal sequence of 2D angiographic training images (140') corresponding to the 3D angiographic training image (130'a), the 2D angiographic training images (140') representing a flow of a contrast agent through the region of interest (120); and
for each of a plurality of 2D angiographic training images (140') in a temporal sequence for a patient, and for each of a plurality of patients:
inputting (S230) the 2D angiographic training image (140') into the neural network (NN1);
generating (S240) a corresponding predicted 3D angiographic image (110') representing the flow of the contrast agent through the region of interest (120);
adjusting (S250) parameters of the neural network (NN1) based on:
i) a value of a first loss function (LF₁) representing a difference between the received 3D angiographic training image (130'a) and the predicted 3D angiographic image (110'); and
ii) a value of a second loss function (LF₂) representing a difference between the inputted 2D angiographic training image (140') and a projection (160) of the predicted 3D angiographic image (110') onto a plane corresponding to a plane of the inputted 2D angiographic training image (140'); and
repeating (S260) the inputting (S230) and the generating (S240) and the adjusting (S250) until a stopping criterion is met.

12. The computer-implemented method according to claim 1, wherein the neural network (NN1) is trained to predict the temporal sequence of 3D angiographic images (110) from the temporal sequence of 2D angiographic images (140), and to constrain the predicted temporal sequence of 3D angiographic images by the volumetric image data (130b); by
receiving (S310), for each of a plurality of patients, a temporal sequence of 3D angiographic training images (130'b) representing a reference flow of the contrast agent through the region of interest (120);
receiving (S320), for each patient, a temporal sequence of 2D angiographic training images (140') corresponding to the temporal sequence of 3D angiographic training images (130b'), the temporal sequence of 2D angiographic training images (140') representing a flow of a contrast agent through the region of interest (120); and
for each of a plurality of 2D angiographic training images (140') in a temporal sequence for a patient, and for each of a plurality of patients:
inputting (S330) the 2D angiographic training image (140') into the neural network (NN1);
generating (S340) a corresponding predicted 3D angiographic image (110') representing the flow of the contrast agent through the region of interest (120);
adjusting (S350) parameters of the neural network (NN1) based on:
i) a value of a first loss function (LF₁) representing a difference between a corresponding 3D angiographic training image from the received temporal sequence of 3D angiographic images (140'b) and the predicted 3D angiographic image (110'); and
ii) a value of a second loss function (LF₂) representing a difference between the inputted 2D angiographic training image (140') and a projection (160) of the predicted 3D angiographic image (110') onto a plane corresponding to a plane of the inputted 2D angiographic training image (140'); and
repeating (S360) the inputting (S330) and the generating (S340) and the adjusting (S350) until a stopping criterion is met.

13. The computer-implemented method according to claim 11 or claim 12, further comprising:
receiving user input indicating an extent of the region of interest (120) in the temporal sequences of 3D angiographic training images (130'a, 130'b) and/or 2D angiographic training images (140'); and wherein:
the value of the first loss function (LF₁) and/or the second loss function (LF₂) is computed only within the indicated extent; or
the value of the first loss function (LF₁) and/or the second loss function (LF₂) is computed by applying a higher weighting to the respective loss functions within the indicated extent, as compared to outside the indicated extent.

14. The computer-implemented method according to claim 11 or claim 12, wherein the respective 3D angiographic training image (130'a) or the temporal sequence of 3D angiographic training images (130'b), represents pre-procedural data generated prior to an insertion of an interventional device into the region of interest, and wherein the temporal sequence of 2D angiographic training images (140') represents intra-procedural data generated during an insertion of the interventional device into the region of interest, and wherein a flow of a contrast agent through the region of interest (120') in the 3D angiographic training image (130'a) or in the temporal sequence of 3D angiographic training images (130'b) is different to the flow of the contrast agent through the region of interest in the temporal sequence of 2D angiographic training images (140').

15. The computer-implemented method according to claim 14, further comprising:
receiving a temporal sequence of 3D angiographic training images representing a flow of the contrast agent through the region of interest (120) during or after an insertion of the interventional device into the region of interest; and
wherein the adjusting (S250, S350) parameters of the neural network (NN1), is based further on:
iii) a value of a third loss function (LF₃) representing a difference between a corresponding 3D angiographic training image representing a flow of the contrast agent through the region of interest during or after an insertion of the interventional device into the region of interest, and the predicted 3D angiographic image (110').
